Europäisches Patentamt

European Patent Office

Office européen des brevets

(19)

(11) Publication number: **0 242 459 B1**

# EUROPEAN PATENT SPECIFICATION

(12)

(45) Date of publication of patent specification: **14.08.91**   (51) Int. Cl.5: **A61K 31/70**

(21) Application number: **86302958.3**

(22) Date of filing: **18.04.86**

(54) **Use of oligosaccharides for promoting the proliferation of bifidobacteria.**

(43) Date of publication of application:
**28.10.87 Bulletin 87/44**

(45) Publication of the grant of the patent:
**14.08.91 Bulletin 91/33**

(84) Designated Contracting States:
**DE FR GB NL**

(56) References cited:

CHEM. PHARM. BULLETIN, vol. 26, no. 11,
1978, pages 3306-3311; K. YAZAWA et al.:
"Oligosaccharides and polysaccharides
specifically utilizable by bifidobacteria"

DICTIONNAIRE VIDAL, 51st edition, 1975,
pages 216,1001,1002; O.V.P., Paris, FR

(73) Proprietor: **Showa Sangyo Co., Ltd.**
**No. 2-1, 2-chome Uchi-Kanda**
**Chiyoda-ku Tokyo(JP)**

(72) Inventor: **Machida, Yoshiaki**
**9-10-1102, 1-chome**
**Matsue, Edogawaku Tokyo(JP)**
Inventor: **Fukui, Fumio**
**2-117, 1-chome Nakadai**
**Naritashi Chibaken(JP)**
Inventor: **Komoto, Takanobu**
**20-2, 2-chome Hinode**
**Funabashi-shi Chibaken(JP)**

(74) Representative: **Lynd, Michael Arthur et al**
**STANLEY, POPPLEWELL, POOLE 57 Lincoln's**
**Inn Fields**
**London WC2A 3LS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates agents for promoting proliferation of intestinal bifidobacteria using isomalto-oligosaccharides as an active substance.

Bifidobacteria which belong to the genus Bifidobacterium have been known as useful microorganisms inhabiting in the human intestines. They have an important role in maintaining human health by preventing the increase of microorganisms which produce harmful substances such as amines and ammonia in the intestines.

The bifidobacteria-proliferating action of sugars such as lactulose, raffinose, stachyose is well-known. Of these sugars, lactulose has been used, in practice, as a food for bifidobacteria. However, these sugars are consumed not only by bifidobacteria but also by microorganisms which produce harmful substances in the intestines. The bifidobacterial-proliferation action of palatinose is also known. Palatinose is an isomer of sucrose. It is also known that fructo-oligosaccharides have a bifidobacteria proliferating action, which fructo-oligosaccharides have molecules consisting of one sucrose of from one to four fructose. They are reported as the useful bifidobacteria proliferation agents since they are utilized selectively by them (Japan Kokai Nos. 91193/1982 and 53334/1984).

Generally, sugars are known to be nutrients for bifidobacteria as well as for noxious microorganisms, and sugars which are utilized only by bifidobacteria are little known. It is not possible to predict from the properties of sugar, such as molecular structure, whether the sugar will be utilized selectively by bifidobacteria or not.

An article in Chem Pharm Bull 26, 3306-3311 (1978) reports that raffinose, stachyose, glucose, lactose, isomaltose and inulin are utilized by bifidobacteria.

The inventors have found a bifidobacteria proliferating action of isomalto-oligosaccharides apart from isomaltose, and, therefore, it is an object of this invention to provide a new bifidobacteria-proliferation promoting substance which is selectively utilized by bifidobacteria and not by noxious microorganisms. To promote the bifidobacteria proliferation, isomalto-oligosaccharides are used as an active substance. The terms "isomalto-oligosaccharides" and "isomalto-sugars" are used herein as a general term for oligo-saccharides having branched structure of Alpha - D - (1 → 6) linkage of glucose unit in the molecules, except that isomaltose is not a new bifidobacteria promoting substance provided by this invention. Isomaltotetraose, panose, ismaltotetraose, isomaltopentaose are typical sugars in this group and may be used along or as a mixture of two or more thereof in this invention. They may be prepared, for example, by reacting a sugar-condensating enzyme such as glucoamylase to an aqueous solution of glucose more than 50% concentration (Japan Patent Application Nos. 245470/1984 and 61248/1985), or by reacting a sugar transfer enzyme such as alpha-clucosidase to an aqueous solution of maltose, preferably accompanying with beta-amylase and a starch-debranching enzyme such as pullulanase (Japan patent application No.58433/1985).

A syrup containing about 40% isomalto-sugar (to solid) can be obtained by the above methods, however it is possible to increase the content over 60% by selecting the conditions appropriately. Sugars other than isomalto-sugars in the syrup are mainly glucose and maltose. A typical composition of isomalto-oligosaccharides in the syrup thus obtained is 20 to 30% of isomaltose, 10 to 15% of panose, isomaltotriose and other trioses, and 5 to 10% of isomaltotetraose and saccharides consisting of four or more glucose units. In accordance with this invention, the new bifidobacteria-promoting proliferation substance in such a syrup is provided by the isomalto-sugars apart from isomaltose.

A syrup containing mainly saccharide consisting of three or more glucose units can also be produced by reacting a sugar transfer enzyme with starch which has been hydrolyzed by starch-debranching enzyme and alpha-amylase (Japan patent application No.53017/1985). The isomalto-sugar composition of the product is 10 to 25% of isomaltose, 20 to 35% of panose, isomaltotriose and other trioses, and 5 to 15 % of isomaltotetraose and saccharides consisting of four or more glucose units.

The isomalto-oligosaccharides syrup thus obtained can further be subjected to, e.g. the following treatments in order to increase the concentration of isomalto-sugars to about 90% or more to the solid.

(1) Concentrating and cooling the syrup slowly to crystallize out glucose etc. and remove them therefrom.

(2) Adding sodium chloride to the syrup to crystallize out a glucose-sodium chloride complex and removing them therefrom.

(3) Adding an organic solvent such as alcohols, acetone, etc. to the syrup to precipitate sugars other than isomalto-oligosaccharides and remove them therefrom.

(4) Removing sugars other than isomalto-oligosaccharides from the syrup by chromatographic separation technique.

(5) Removing sugars other than isomalto-oligosaccharides from the syrup by the yeast fermentation.

The sugar composition of concentrated isomalto-oligosaccharides thus obtained may be as follows:

Table 1

| Principal component | Isomaltose | Isomalto- sugar comprising three or more glucose units |
|---|---|---|
| Isomaltose | 40~70(%) | 25~35(%) |
| Panose, Isomaltotriose and other trioses | 15~30 | 40~50 |
| Isomaltotetraose and saccharides consist of four or more glucose units | 10~20 | 15~20 |

These isomalto-sugars syrup may be further treated, if necessary, by gel-filtration or ion exchange resin column to fractionate the sugars by the numbers of glucose units. Fractions isomalto-disaccharide, mainly isomaltose, isomalto-trisaccharide, mainly panose and isomaltotriose, and isomalto-tetrasaccharide mainly isomaltotetraose, can be obtained separately, and they are also active as a bifidobacteria proliferating agent.

The isomalto-sugar syrup or the separated fractions may further be powdered by well-known method such as spray drying, adsorbing on a carrier, etc.

The bifidobacteria proliferation promoting action of the isomalto-oligosaccharides provided by the invention was examined in comparison with isomaltose and other sugars, as follows:

Several kinds of microorganisms including genus bifidobacterium were incubated in a sterilized culture media consisting of peptone-yeast-Fildes solution (hereinafter referred to as "PYF medium") each of which respectively 0.5% concentration of sugars tested were added.

1. Culture medium

The composition of PYF medium (pH 7.2) was as follows:

| | |
|---|---|
| Pancreatic digest of casein (BBL Trypticase peptone made by Becon Dickinson and Company) | 10 grams |
| Yeast extract (made by Difco Company) | 5 grams |
| Fildes solution | 40 millilitres |
| Salts solution | 40 millilitres |

| | |
|---|---|
| $CaCl_2$ (anhydrous) | 0.2 grams |
| $MgSO_4$ | 0.2 |
| $K_2HPO_4$ | 1.0 |
| $KH_2PO_4$ | 1.0 |
| $NaHCO_3$ | 10.0 |
| NaCl | 2.0 |
| Pure water | 1000 millilitres |

| | |
|---|---|
| L-Cysteine .$HCl.H_2O$ | 0.5 grams |
| Pure water | 920 millilitres |

Fildes solution was prepared by mixing the following components, digesting the mixture for one night in a bath maintained at 55° C, adding 12 millilitres 20% aqueous solution of NaOH, and adjusting the pH to 7.6 with NaOH.

| | |
|---|---|
| Physiological saline (0.85% aqueous solution of NaCl) | 150 millilitres |
| Concetrated hydrochloric acid | 6 millilitres |
| Horse blood | 50 millilitres |
| Pepsine, 1/10000 aqueous solution (Difco Co.) | 1 gram |

2. Method and result

Fresh microorganisms incubated by agar plate culture media were inoculated $10^8$ cfu (colony formation unit) per each test tube of PYF medium, in which sugars to be examined were added at 0.5% concentration. They were incubated at 37° C for 48 hours under anaerobic conditions by the steelwool method. The absorbance of the resultant liquid at 650 nanometers was determined, and the consumption of tested sugars by the microorganisms were calculated by the following equation.

$$RG = \frac{OD\ for\ tested\ sugar\ -\ OD\ for\ PYF\ medium}{OD\ for\ glucose\ -\ OD\ for\ PYF\ medium} \times 100$$

$$RG : rate\ of\ growth \qquad OD : optical\ density$$

Results are shown in Table 2, in which the signs represent the following RG values.

$+ + : RG \geq 75 \quad + : 50 \leq RG < 75 \quad \pm : 25 \leq RG < 50 \quad - : RG < 25$

4

## Table 2

| Microorganisms | gluc-ose | malt-ose | lact-ose | raffin-ose | isomalto-oligosaccharides | | | |
|---|---|---|---|---|---|---|---|---|
| | | | | | 90% mixt.* | isomalt-ose | tri-ose** | tetra-ose*** |
| Bifidobacterium adolescentis E298 | ++ | ++ | ++ | -+ | ++ | +- | ++ | -+ |
| Bifidobacterium infantis I-10-5 | +- | +- | ++ | -- | +- | ++ | +- | +- |
| Bifidobacterium breve S1 | ++ | ++ | ++ | ++ | -- | +- | ++ | +- |
| Bifidobacterium longum E194 | +- | +- | ++ | -+ | + | +- | ++ | -+ |
| Lactobacillus acidophilus ATCC4356 | +- | + | -- | - | - | -- | -- | - |
| Clostridium paraputrificum | ++ | ++ | +- | - | - | - | - | - |
| Clostridium SP2 | +- | +- | ± | - | - | - | - | - |
| Bacteroides vulgatus | +- | ++ | +- | + | - | ± | - | - |
| Bacteroides ovatus | +- | ++ | ++ | + | - | - | - | - |
| Escherichia coli | ++ | ++ | ± | ++ | - | - | - | - |
| Peptostrepto-coccus anaerobius X-36 | - | - | - | ++ | - | - | - | - |
| Fusobacterium varium VI-43 | +- | - | - | - | - | - | - | - |

* The 90% mixture contains 90% of isomalto-oligosaccharides to the solid and the component is as follows:

| | |
|---|---|
| Isomaltose | 50% |
| Isomalto-trisaccharides (Isomaltotriose,Panose,etc) | 25% |
| Other isomalto-sugars | 15% |
| Sugars other than isomalto-sugars | 10% |

(mainly glucose and maltose)

In this 90% mixture and other mixtures and syrups containing isomaltose, the isomalto-oligosaccharides apart from isomaltose aretheisomalto-oligosaccharides in accordance with this invention.

```
**  Over 85% (to the solid) of triose mainly panose and

    isomaltotriose.

*** Over 85% (to the solid) of tetraose mainly isomalto-

    tetraose.
```

The isomalto-sugars are consumed by the microorganisms of genus bifidobacterium as well as the other sugars. However, most microorganisms other than bifidobacteria do not consume the isomalto-sugars, as is seen from Table 2, and thus, it is apparent that the isomalto-sugars are consumed selectively by bifidobacteria.

Raffinose is not consumed by some of the microorganisms such as the genus Clostridium or genus Fusobacterium, while other sugars such as glucose and lactose are consumed by all microorganisms. However, raffinose is consumed by Escherichia coli and the genus Bacteroides which are microorganisms that produce harmful substances in the intestine, and the selectivity for bifidobacteria is apparently lower than that of isomalto-sugars.

The isomalto-sugars may be provided as a bifidobacteria-proliferating agent, using the isomalto-sugars singly or as a mixture of two or more thereof, with foodstuffs or a raw material thereof, or with pharmaceutically acceptable diluent in any suitable form, such as powder, granule, tablet, sugar-coated tablet, capsule, suspension, solution, emulsion, ampoule etc.

As an active ingredient, the isomalto-sugars may be present in an amount of from 0.1 to 100% by weight in the proliferating agent which is taken orally or parenterally in this invention.

The amount for the oral intake of the proliferating agent is generally within the range between 0.01 and 2.0 grams per kg of the body weight per day, preferably 0.1 to 1.5 g/kg/day, although this will vary depending on the individual.

Bifidobacteria may be admixed in the proliferating agent in order to increase the proliferation-promoting action. For this purpose, dried cells of the microorganisms belong to the genus Bifidobacterium may be used in an amount of from about 30 to 70 parts by weight to 70 to 30 parts by weight of the isomalto-oligosaccharides solid.

Vitamins, e.g. pantetheine, pantothenic acid, and riboflavin are also known to be essential substances for the growth of Bifidobacterium, and therefore, their use together with the isomalto-oligosaccharides is also effective to increase the quantity of Bifidobacterium in the intestines. Suitable amount may be from about 1/100 to 1/300 of the weight of the isomalto-oligosaccharides.

EXAMPLE 1

Bread was made by a normal process from the following composition:

| Composition | Control | Test |
|---|---|---|
| Wheat flour | 500 g | 500 g |
| Sucrose | 100 | 50 |
| 55% fructose syrup* | 33 | 33 |
| Isomalto-sugar** | 0 | 6.6 |
| Whole egg | 50 | 50 |
| Shortening | 30 | 30 |
| Dried yeast | 10 | 10 |
| Yeast food | 0.5 | 0.5 |
| Salt | 3 | 3 |
| Water | 217 | 201 |

*moisture 25%, *Sunfruct* made by Sanmatsu Kogyo Co.

** moisture 24.2% and about 50% of isomalto-oligosaccharides to the solid,

Dough was prepared by the following steps:
1) Yeast was activated prior to use by mixing warm water to the mixture of dried yeast and a small amount of sucrose.
2) Sugars including isomalto-sugar syrup, egg, yeast food, salt and half of the flour were mixed in a mixing bowl, and the mixture kneaded at 160 rpm for 1 minute.
3) Yeast was added and the mixture kneaded at 160 rpm for 3 minutes.
4) The rest of flour was added and the mixture kneaded at 160 rpm for 1 minute and then at 230 rpm for 3 minutes.
5) Shortening was added and the mixture kneaded at 230 rpm for 4 minutes to obtain a dough.
 Fermentation and baking were carried out as follows:
1) The dough was placed in a thermo-hygrostat of 29°C temperature and 90 to 100% relative humidity for 2 hours.
2) The dough was punched (degassed) and placed in the same conditions for 1 hour.
3) The dough was punched again and further fermented in the same conditions for 20 minutes.
4) The dough was cut into pieces each 50 grams.
5) Bench at 30 to 31°C for 20 minutes.
6) Proof at 36 to 37°C for 60 minutes.
7) Bake at 180°C for 10 minutes.
 The moisture content of the bread used isomalto-oligosaccharides ("test") was stable during the preservation and the staling was considerably retarded. Tast, texture of crumb, etc. were also excellent compared to the bread of the "control".

EXAMPLE 2

Fifty parts of spray dried isomalto-oligosaccharides containing about 90% isomalto-sugar were admixed

7

with 5 parts of magnesium stearate, 25 parts of corn starch and 20 parts of lactose and made into tablet form. The composition of isomalto-oligosaccharides was as follows:

Isomaltose        : 28%
Panose and Isomaltriose   : 45
Isomaltotetraose and others        : 17

EXAMPLE 3

1) The bread prepared in Example 1 were fed to twenty male and female volunteers aged between 25 and 35 years old. The volunteers were divided into two groups, and the bread "control" was fed to the first group and the bread "test" to the other. The daily intake of isomalto-oligosaccharides was about 6 grams for the "test" group, and the feeding continued for 4 weeks. The faeces samples were collected from the twenty persons at the beginning and the final days of the period, and the numbers of microorganisms in the samples were determined. It was observed that the number of microorganisms belong to the genus Bifidobacterium increased about 100 times comparing the beginning and the final days at the "test" group, while the number was unchanged at the "control" group. The numbers of the microorganisms other than bifidobacteria such as genus Clostridium, Escherichia coli, etc. of the final day were relatively lower than the beginning in the "test" group.

2) The tablets prepared in Example 2 were fed to ten male volunteers ages between 65 and 80 years old, daily 20 grams of isomalto-sugar for 60 days. The numbers of microorganisms in the faeces collected from all the members were determined every 5 days from the beginning of the feeding. An increase in the number of bifidobacteria during the whole period was observed for 7 persons, and for three of these the increase was considerableof the other three volunteers. An increase in the numbers was not observed for one while a very slight increase was observed for two.

## Claims

1. Isomalto-oligosaccharides with the exception of isomaltose, for use as agents for selectively promoting the proliferation of the microorganisms of the genus Bifidobacterium in the human intestines.

2. Isomalto-oligosaccharides for the use according to claim 1, characterized in that the isomalto-oligosaccharide comprises panose.

3. Isomalto-oligosaccharides for the use according to claim 1 or 2 characterized in that the isomalto-oligosaccharide comprises isomaltotriose.

4. Isomalto-oligosaccharides for the use according to claim 1, 2 or 3, characterized in that the isomalto-oligosaccharide comprises isomaltotetraose.

5. Isomalto-oligosaccharides for the use according to claim 1, characterized in that the isomalto-oligosaccharide comprises a mixture of at least two kinds of sugars selected from panose, isomaltotriose, and isomaltotetraose.

6. Isomalto-oligosaccharides for the use according to any one of claims 1 to 5, characterized in that the agent further comprises microorganisms of the genus Bifidobacterium, in an amount of from 30 to 70 parts by weight of cell to 70 to 30 parts by weight of the isomalto-oligosaccharide.

7. Isomalto-oligosaccharides for the use according to claim 6, characterized in that said microorganisms are present as dried cells.

8. Isomalto-oligosaccharides for the use according to any one of claims 1 to 7, characterized in that the agent further comprises vitamins in an amount of from 1/100 to 300 of vitamin to the weight of isomalto-oligosaccharides.

9. Isomalto-oligosaccharides for the use according to claim 8, characterized in that the vitamin is selected from at least one of pantheteine, pantothenic acid and riboflavin.

10. Isomalto-oligosaccharides for the use according to any of claims 1 to 9, characterized in that it is in the form of a human foodstuff.

11. Isomalto-oligosaccharides for the use according to any of claims 1 to 10, together with isomaltose.

**Revendications**

1. Isomalto-oligosaccharides, à l'exception de l'isomaltose, pour un usage comme agents pour promouvoir sélectivement la prolifération des microorganismes du genre Bifidobactérium dans l'intestin humain.

2. Isomalto-oligosaccharides pour l'usage suivant la revendication 1, caractérisés en ce que l'isomaltooligosaccharide comprend du panose.

3. Isomalto-oligosaccharides pour l'usage suivant la revendication 1 ou 2, caractérisés en ce que l'isomaltooligosaccharide comprend de l'isomaltotriose.

4. Isomalto-oligosaccharides pour l'usage suivant la revendication 1, 2 ou 3, caractérisés en ce que l'isomalto-oligosaccharide comprend de l'isomaltotétraose.

5. Isomalto-oligosaccharides pour l'usage suivant la revendication 1, caractérisés en ce que l'isomaltooligosaccharide comprend un mélange d'au moins deux sortes de sucres choisis parmi le panose, l'isomaltotriose et l'isomaltotétraose.

6. Isomalto-oligosaccharides pour l'usage suivant l'une quelconque des revendications 1 à 5, caractérisés en ce que l'agent comprend en outre des microorganismes du genre Bifidobactérium, en quantité allant de 30 à 70 parties en poids de cellules à 70 à 30 parties en poids de l'isomalto-oligosaccharide.

7. Isomalto-oligosaccharides pour l'usage suivant la revendication 6, caractérisés en ce que lesdits microorganismes sont présents sous forme de cellules désséchées.

8. Isomalto-oligosaccharides pour l'usage suivant l'une quelconque des revendications 1 à 7, caractérisés en ce que l'agent comprend en outre des vitamines en quantité allant de 1/100 à 300 de vitamine par rapport au poids d' isomalto-oligosaccharides.

9. Isomalto-oligosaccharides pour l'usage suivant la revendication 8, caractérisés en ce que la vitamine est choisie parmi au moins une des panthétéine, acide pantothénique et riboflavine.

10. Isomalto-oligosaccharides pour l'usage suivant l'une quelconque des revendications 1 à 9, caractérisées en ce qu'ils sont sous forme d'un produit alimentaire humain.

11. Isomalto-oligosaccharides pour l'usage suivant l'une quelconque des revendications 1 à 10 en association avec de l'isomaltose.

**Patentansprüche**

1. Isomalto-Oligosaccharide mit Ausnahme von Isomaltose zur Verwendung als Mittel zur selektiven Förderung der Proliferation der Mikroorganismen der Gattung Bifidobakterium in den menschlichen Eingeweiden.

2. Isomalto-Oligosaccharide zur Verwendung nach Anspruch 1, **dadurch gekennzeichnet,** daß die Isomalto-Oligosaccharide Panose umfassen.

3. Isomalto-Oligosaccharide zur Verwendung nach Anspruch 1 oder 2, **dadurch gekennzeichnet,** daß die Isomalto-Oligosaccharide Isomaltotriose umfassen.

4. Isomalto-Oligosaccharide zur Verwendung nach Anspruch 1, 2 oder 3, **dadurch gekennzeichnet,** daß die Isomalto-Oligosaccharide Isomaltotetraose umfassen.

5. Isomalto-Oligosaccharide zur Verwendung nach Anspruch 1, **dadurch gekennzeichnet,** daß die Isomalto-Oligosaccharide eine Mischung von wenigstens zwei Arten von Zuckern umfassen, welche aus Panose, Isomaltotriose und Isomaltotetraose ausgewählt sind.

6. Isomalto-Oligosaccharide zur Verwendung nach wenigstens einem der Ansprüche 1 bis 5, **dadurch gekennzeichnet**, daß das Mittel ferner Mikroorganismen der Gattung Bifidobakterium in einer Menge von 30 bis 70 Gewichtsteilen der Zelle zu 70 bis 30 Gewichtsteilen der Isolmalto-Oligosaccharide umfaßt.

7. Isomalto-Oligosaccharide zur Verwendung nach Anspruch 6, **dadurch gekennzeichnet**, daß die Mikroorganismen als getrocknete Zellen vorliegen.

8. Isomalto-Oligosaccharide zur Verwendung nach wenigstens einem der Ansprüche 1 bis 7, **dadurch gekennzeichnet**, daß das Mittel ferner Vitamine in einer Menge von 1/100 bis 300 der Vitamine zum Gewicht der Isomalto-Oligosaccharide umfaßt.

9. Isomalto-Oligosaccharide zur Verwendung nach Anspruch 8, **dadurch gekennzeichnet**, daß das Vitamin aus wenigstens einem von Panthetein, Pantothensäure und Riboflavin ausgewählt ist.

10. Isomalto-Oligosaccharide zur Verwendung nach wenigstens einem der Ansprüche 1 bis 9, **dadurch gekennzeichnet**, daß sie in der Form von Nahrungsmittel für den Menschen vorliegen.

11. Isomalto-Oligosaccharide zur Verwendung nach wenigstens einem der Ansprüche 1 bis 10 zusammen mit Isomaltose.